# EUROPEAN PATENT APPLICATION

(11) **EP 2 409 682 A1**
(43) Date of publication of application: **25.01.2012**
(21) Application number: 10007453.3
(22) Date of filing: 19.07.2010
(51) Int. Cl.: A61K 8/64, A61Q 11/00, A61K 8/25

(54) **Hydroxyapatite-binding nano- and microparticles for caries prophylaxis and reduction of dental hypersensitivity**

(71) Applicant: MÜLLER, Werner E. G., 65203 Wiesbaden (DE); Wiens,, Matthias, Dr., 55270 Essenheim (DE)
(72) Inventor: MÜLLER, Werner E. G., 65203 Wiesbaden (DE); Wiens,, Matthias, Dr., 55270 Essenheim (DE)
(74) Representative: Krauss, Jan

(57) **Abstract**

The invention relates to the application of novel oligopeptide-functionalized nanoparticles or microparticles, which bind to hard tissue (hydroxyapatite) of tooth enamel and dentin for sealing of pits, fissures, and dentinal tubules of teeth, for caries prophylaxis, for delivery of drugs, dyes, bioactive compounds, and to reduce dental hypersensitivity.

## Description

The present invention relates to nano-/microparticles functionalized with a hydroxyapatite-binding oligopeptide (tagged nano/microparticles), which can be used for sealing of dental pits, fissures, and dentinal tubules, to prevent caries formation, and to reduce dental hypersensitivity (dentin hypersensitivity).

### Background of the invention

Human teeth are composed of four tissues: enamel, dentin, dental pulp, and cementum. Enamel is the hardest mineralized tissue in the body; it almost entirely consists of carbonated hydroxyapatite (approx. 95 wt.%). The enamel crystals are formed on an organic matrix, which is produced by the ameloblasts. Crystal formation is mainly controlled by the enamel matrix proteins amelogenin, ameloblastin, and enamelin. The amelogenins are involved in the initial mineralization process; they build the lattices that guide the spacing and the orientation of enamel crystallites. Ameloblastin controls the elongation of the enamel crystals and serves as a sheet protein to stabilize the Tomes processes to the enamel matrix. Enamelin binds to enamel crystallites. The enamel crystals are tightly packed to rod-like structures; the long axis of each enamel rod is perpendicular to the underlying dentin, which supports the enamel layer. Dentin is less mineralized. It comprises only ca. 70 wt.% carbonated hydroxyapatite. Dentin contains canals (dentinal tubules) with diameters in the micron range. These tubules radiate outward to the dentin/enamel border and may cause, by exposure of nerves located at the pulpal aspect, dentin hypersensitivity. This sensation can range from irritation to intense, shooting pain. In addition, dentin permeability facilitates tooth decay.

Generally, the high content of carbonated hydroxyapatite renders teeth susceptible to demineralization and is responsible for the development of dental caries (tooth decay). Caries is one of the most common diseases worldwide. It is caused by acid-producing bacteria, which metabolize carbohydrates. Two groups of bacteria are responsible for initiating caries, *Streptococcus mutans* and *Lactobacillus.* Pits, fissures, and grooves in the enamel layer facilitate caries formation. As demineralization continues, underlying dentin becomes affected. Consequently, enamel is unable to compensate for its brittleness, breaks away, and ultimately enhances tooth decay even further.

Previous strategies of the inventors for prophylaxis of caries by coating teeth (sealing of tooth pits and fissures) with a silica layer solely base on the application of an enzyme (silicatein), which binds to the dental surface and, subsequently, catalyses silica formation. Silicatein and its application have been patented (German Patent No. DE10037270**,** European Patent No. EP1320624, United States Patent No. US7,169,589B2, Chinese Patent No. ZL 01813484.X**,** New Zealand Patent No. 523474, Australia Patent No. 2001289713**.** Silicatein-mediated synthesis of amorphous silicates and siloxanes and their uses. Inventors: Müller WEG, Lorenz A, Krasko A, Schröder HC; national phases: N020030407; Japan No. 2002-516336; Canada No. 2,414,602). Besides silicatein-α (above patents), the use of further silicateins has been applied for a patent by the inventors, including silicatein-β (patent application: DE10352433.9**.** Enzym- und Template-gesteuerte Synthese von Silica aus nicht-organischen Siliciumverbindungen sowie Aminosilanen und Silazanen und Verwendung. Inventors: Schwertner H, Müller WEG, Schröder HC), and four silicatein isoforms of a freshwater sponge (silicatein-a1-4; DE102006001759.5. Kontrollierte Herstellung von Silber- und Gold-Nanopartikeln und Nanokristallen definierter Größe und Form durch chirale Induktion mittels Silicatein. Inventors: Tremel W, Tahir MN, Müller WEG, Schröder HC), or patented by others (PCT/US99/30601. Methods, compositions, and biomimetic catalysts, such as silicateins and block copolypeptides, used to catalyze and spatially direct the polycondensation of silicon alkoxides, metal alkoxides, and their organic conjugates to make silica, polysiloxanes, polymetallo-oxanes, and mixed poly(silicon/metallo)oxane materials under environmentally benign conditions. Inventors: Morse DE, Stucky GD, Deming TD, Cha J, Shimizu K, Zhou Y).

Furthermore, the inventors have reported that silica shows an osteoblast-stimulating activity. Thus, formation of mineralized calcium phosphate nodules of human bone-forming cells significantly increases when the cells are grown on surfaces coated with enzymatically synthesized biosilica (Schröder HC, Boreiko O, Krasko A, Reiber A, Schwertner H, Müller WEG. J Biomed Mater Res Part B: Appl Biomater 75B, 387-392, 2005). Silica also promotes the expression of genes that are crucially involved in enamel formation: amelogenin, ameloblastin, and enamelin (Müller WEG, Boreiko A, Wang X, Krasko A, Geurtsen W, Custódio MR, et al. Calcif Tissue Int 81, 382-393, 2007).

The following publications are regarded as relevant for the application of silica in dentistry. These publications describe the application of enzymatically formed silica (biosilica), but not the application of the synthetic hydroxyapatite-binding, oligopeptide-functionalized silica particles herein described by the inventors.
1. Schröder HC, Boreiko A, Krasko A, Reiber A, Schwertner H, Müller WEG. Mineralization of SaOS-2 cells on enzymatically (Silicatein) modified bioactive osteoblast-stimulating surfaces. J Biomed Mater Res B Appl Biomater 75B:387-392, 2005.
2. Müller WEG, Boreiko A, Wang XH, Krasko A, Geurtsen W, Custódio MR, Winkler T, Lukid-Bilela L, Link T, Schröder HC. Morphogenetic activity of silica and biosilica on the expression of genes, controlling biomineralization using SaOS-2 cells. Calcif Tissue Int 81:382-393, 2007.
3. Wiens M, Bausen M, Natalio F, Link T, Schlossmacher U, Müller WEG. The role of the silicatein-alpha interactor silintaphin-1 in biomimetic biomineralization. Biomaterials 30:1648-1656, 2009.

The application of enzymatically formed silica (biosilica) in biomedicine and dentistry is described in the following patent applications. These patent applications do not include application of the synthetic hydroxyapatite-binding, oligopeptide-functionalized silica particles as described in the present patent application:
DE 102004021229.5**;** EP 2005004738**;** US 11579020**;** JP 2007509992**;** CA2565121**.** Enzyma**t**ic method for producing bioactive, osteoblast-stimulating surfaces and use thereof. Inventors: Müller WEG, Schwertner H, Schröder HC. US 60839601**;** EP 2007007363. Biosilica-adhesive protein nano-composite materials: synthesis and application in dentistry. Inventors: Müller WEG, Schröder HC, Geurtsen WK.
PCT/US2009/005302**.** Compositions, oral care products and methods of making and using the same. Inventors: Miller J, Höfer H, Geurtsen W, Lücker P, Wiens M, Schröder HC, Müller WEG. European Patent application EP 10167744.1. Injectable material and material to be used as drug or food supplement for prophylaxis or treatment of osteoporosis. Inventors: Müller WEG, Wang X, Wiens M.

### Summary of the invention

Previous results of the inventors revealed that enzymatically formed silica (biosilica) can be used for dental applications, for example as a protective dental layer to prevent caries formation. Thereby binding of silica to the hydroxyapatite surface is achieved through the protein (silicatein) component. The application of enzymatically formed silica may be hampered by the fact that formulations containing such enzymatically formed biosilica, or being able to enzymatically form biosilica, contain a protein (i.e., the enzyme). This may be of advantage but also of disadvantage for certain dental applications. In addition, the composition of some dental formulations may inhibit or lower the enzyme activity. Silica particles that are able to directly bind to dental hydroxyapatite, without a protein/silicatein component, have not yet been described. The inventors succeeded to develop, for the first time, such particles. They have demonstrated that silica particles (micro- or nanoscale) that had been modified using a method based on the inorganic chemical functionalization with oligopeptides have high binding affinity to hydroxyapatite. They have further demonstrated that such hydroxyapatite-binding, oligopeptide-functionalized silica nano- and microparticles (also termed "tagged silica nano- and microparticles") not only coat dental surfaces but are also able to completely fill and seal smallest pits and ultrathin fissures in tooth enamel and dentin, without prior clumping or aggregation. In addition, the inventors have discovered that the tagged silica particles unexpectedly target to and seal dentinal tubules: Depending on the particles' size, the tubules are deeply filled (nanoparticles) or their opening is occluded (microparticles). Therefore, hydroxyapatite-binding, oligopeptide-functionalized silica nano-/microparticles have advantageous properties, rendering them particularly suitable for dental applications, such as sealant for dental pits and fissures in caries prophylaxis or as sealant for dentinal tubules in treatment and prophylaxis of dentine hypersensitivity. Additionally, when porous nano- and microparticles are employed, they might be loaded with bioactive compounds (e.g., antiemetics, antibiotics, caffeine, nicotine) or cosmetics (e.g., dyes) used in dentistry and dental care/- hygiene prior to their application to teeth. Following immobilization of the loaded particles on dental hydroxyapatite, their load will be released time-dependently. Due to their chemical functionalization, which confers high hydroxyapatite-binding affinity, the silica particles are superior to similar approaches that use Bioglass or synthetic hydroxyapatite. Bioglass or synthetic hydroxyapatite do not or only weakly bind to dental hydroxyapatite.

### Description of the invention

This invention concerns nano- or microparticles (herein also "nano-/microparticles") functionalized with a hydroxyapatite-binding oligopeptide (tagged nano- or microparticles) which can be preferably used for sealing of dental pits, fissures, and dentinal tubules, to prevent caries formation and to reduce dental hypersensitivity (dentin hypersensitivity). The nano-/microparticles can consist of a mineral, in particular silica (amorphous silicon oxide), zeolite (microporous aluminosilicates) or periodic mesoporous organosilicas (PMOs).

The oligopeptide used for functionalization of the nano-/microparticles preferably consists of amino acids, which carry one or two carboxylic acid groups in their side chains, such as glutamic acid, aspartic acid, or gamma-carboxyglutamic acid, or - alternatively - amino acids, which carry an amino group or an amino group and a hydroxyl group in their side chains, such as lysine and hydroxylysine. The chain length of these oligopeptides preferably ranges from 3 to 18 amino acid residues. The preferred chain length is 8 amino acid residues. The amino acids are linked to a terminal hydroxylated amino acid residue, preferable an N-terminal serine residue, required for binding to the functionalized nano-/microparticles. An aminoalkanoic acid spacer with a chain length of 2 to 16 carbon residues, preferably a C-terminal 6-aminohexanoic acid (Ahx), is preferably used as spacer that is required for covalent binding of suitable dyes, preferably fluorescent dyes (e.g., FITC).

The invention will now be described further in the following preferred examples, nevertheless, without being limited thereto. For the purposes of the present invention, all references as cited herein are incorporated by reference in their entireties. In the Figures and sequence listing,

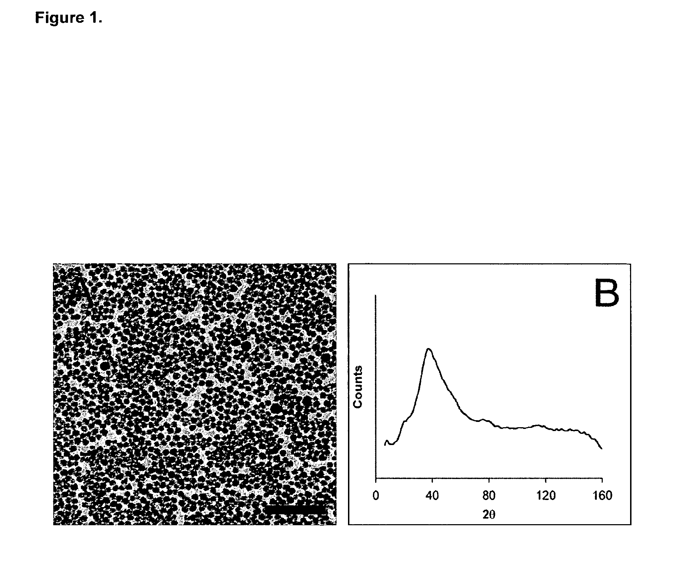

**Figure 1** shows transmission electron microscope (TEM) images of silica nanoparticles (**A**). Scale bar: 100 nm; and corresponding X-ray diffraction pattern indicating that these nanoparticles are amorphous (**B**).

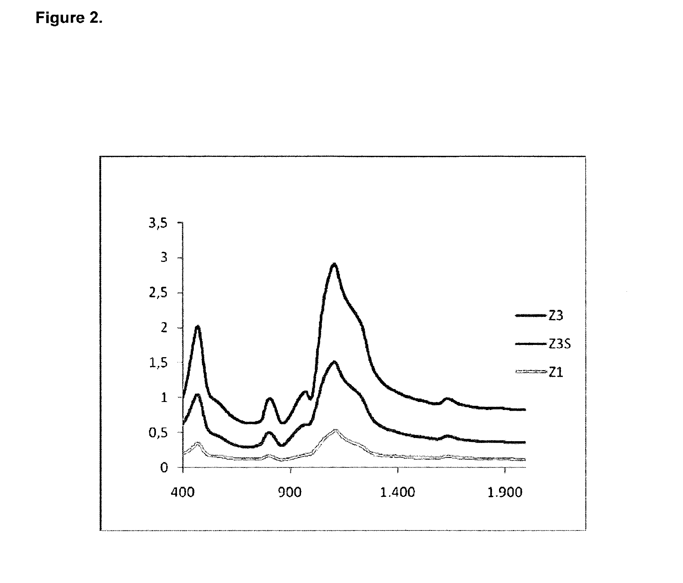

**Figure 2** shows FTIR-ATR spectra of amorphous silica nanoparticles. Green spectrum, non-functionalized; blue spectrum, silyl chloride functionalized particles upon incubation with oligopeptide (EEEEEEEE); red spectrum, silyl chloride functionalized particles upon incubation with serine-oligopeptide (SEEEEEEEE). In the graph, the y-axis depicts the transmittance (%) and the x-axis the wavenumber (cm⁻¹).

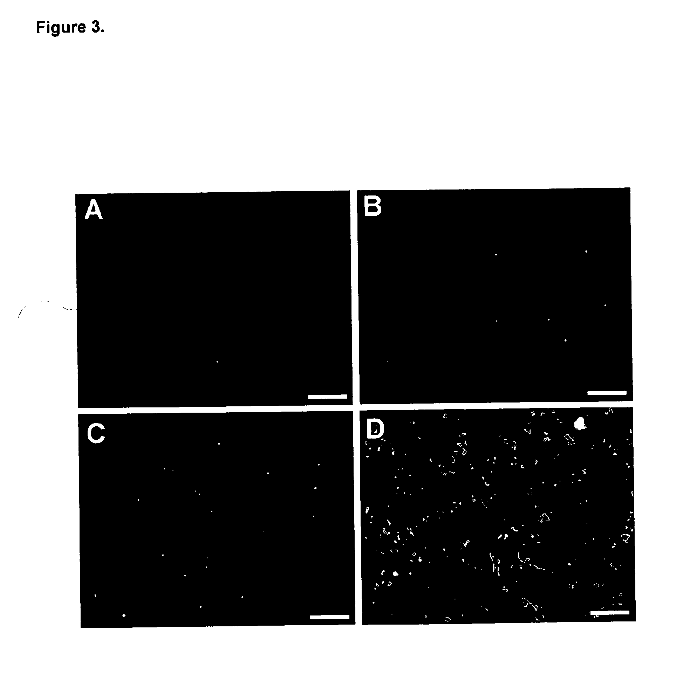

**Figure 3** shows fluorescent microscopic images of silica nanoparticles (20 nm) that had been incubated with FITC-labeled oligopeptides. Silica nanoparticles without previous SOCl₂ treatment, incubated with EEEEEEEE-Ahx-K-FITC (**A**) and SEEEEEEEE-Ahx-K-FITC (**B**), were used as control and show almost complete absence of fluorescent signals. Following incubation of silica nanoparticles, silyl chloride functionalized, with EEEEEEEE-Ahx-K-FITC weak fluorescence signals can be observed due to unspecific and non-covalent surface adsorption (**C**). In contrast, incubation with SEEEEEEEE-Ahx-K-FITC elicited strong and abundant fluorescence signals due to the covalent binding of the serine's hydroxyl group to the functionalized surface (**D**). Scale bar: 10 µm (A-C), 1 µm (D).

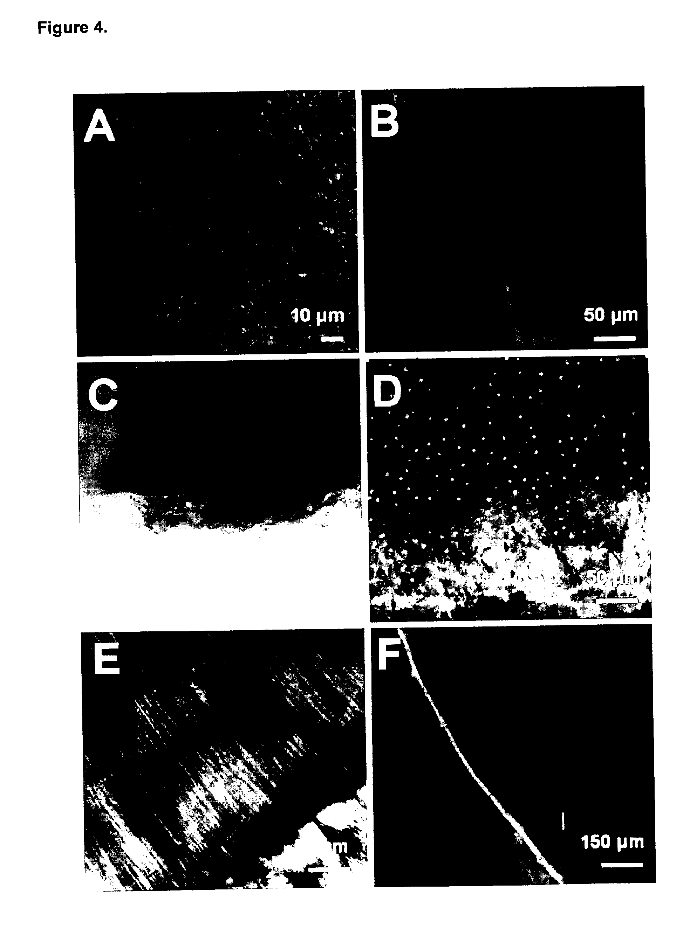

**Figure 4** shows confocal laser scanning microscopic images of dental slices (dental hydroxyapatite), incubated with FITC-labeled silica nanoparticles (20 nm) for 15 min at room temperature. (**A**) Dental slice incubated with silica nanoparticles, tagged with FITC-labeled EEEEEEEE; cross section. A weak fluorescent signal can be observed due to unspecific binding of residual particles that had not been washed away. (B,C) Dental slice incubated with silica nanoparticles, tagged with FITC-labeled EEEEEEEE; longitudinal section. The tooth edge is shown.. No fluorescence can be observed. (**D**) Dental slice incubated with SEEEEEEEE-Ahx-K-FITC tagged silica nanoparticles; cross section. Strong fluorescent signals depict the surface coating and filling of dentinal tubules with silica nanoparticles. (**E**) Dental slice incubated with SEEEEEEEE-Ahx-K-FITC tagged silica nanoparticles; longitudinal section. The image shows the surface coating and filling of dentinal tubules with silica nanoparticles. (**F**) Dental slice incubated with SEEEEEEEE-Ahx-K-FITC tagged silica nanoparticles; longitudinal section. The image shows the fluorescently-tagged coating of the dental surface.

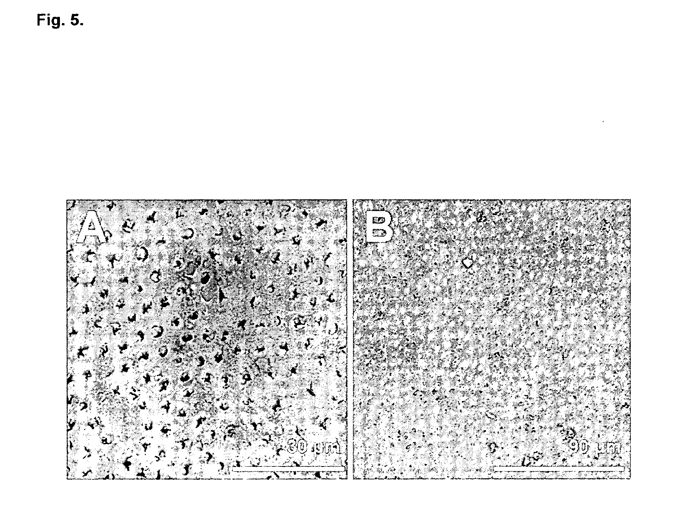

**Figure 5** shows scanning electron micrographs of dental slices (cross sections), incubated with silica nanoparticles. (**A**) Dental slice, incubated with EEEEEEEE-tagged silica nanoparticles. The openings of the cross-sectioned dentinal tubules are plainly visible (**B**) Dental slice, incubated with SEEEEEEEE-tagged silica nanoparticles. Almost all tubules' openings are completely filled with the tagged nanoparticles.

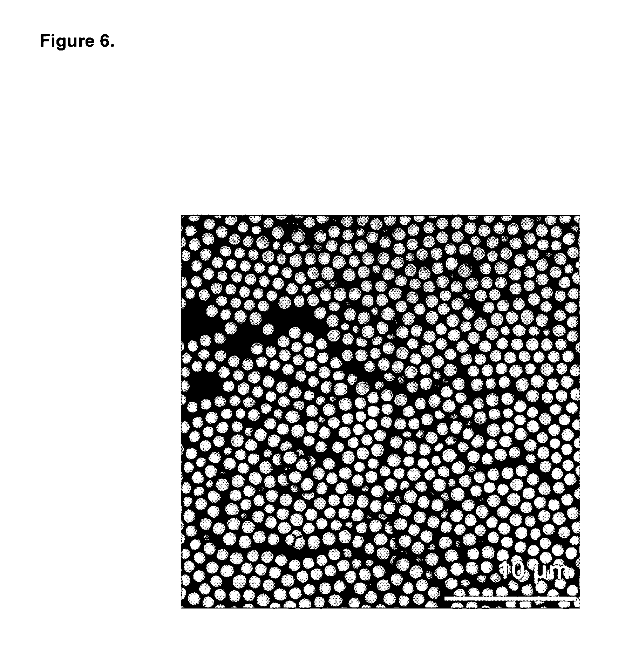

**Figure 6** shows scanning electron micrograph of silica microparticles (diameter: 1 µm).

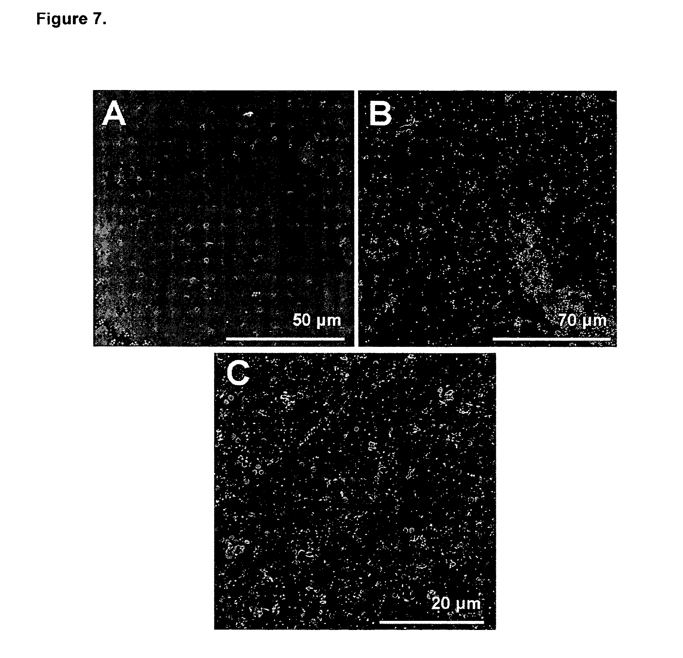

**Figure 7** shows scanning electron micrographs of dental slices (cross sections), incubated with silica microparticles. (**A**) Dental slice with EEEEEEEE-tagged silica microparticles. Only few particles can be observed, non-specifically bound to the surface. (**B-C**) Dental slice with SEEEEEEEE-tagged silica microparticles. C, higher magnification of B. Unexpectedly, the particles target the dentinal tubules' openings. Almost all openings of the cross-sectioned dentinal tubules are occluded (due to the particles' round shape, the occlusion is not complete), usually by 1-3 silica microparticles.

SEQ ID Nos. 1 and 2 show the SEEEEEEEE and EEEEEEEE oligopeptide-linkers.

The examples described in the following in detail concern nano-/microparticles, which have been functionalized with an oligopeptide comprising the following amino acid sequences (single letter amino acid code): SEEEEEEEE (SE₈) or SEEEEEEEE-Ahx-K-FITC (SE₈-FITC). The terminal serine residue (S) contributes the hydroxyl group required for the covalent binding to the silyl chloride-functionalized nano-/microparticles. 6-aminohexanoic acid (Ahx) was used as spacer; the lysine (K) residue provides an ε-amino group, which has been used for labeling of the peptide with a fluorescent dye such as fluorescein isothiocyanate (FITC).

### Morphology of the particles and XRD

In the experiment shown in Figure 1, silica nanoparticles (nanospheres) with a narrow size distribution of 20 nm were analyzed. Figure 1A shows a transmission electron microscopy (TEM) overview of the silica nanospheres. These particles were also analyzed by high-resolution transmission electron microscopy (HRTEM) showing an amorphous structure. Figure 1B displays a XRD (X-ray powder diffraction in θ/2θ reflection geometry) pattern typical for amorphous structures, confirming the HRTEM analysis.

### FT-IR ATR analyses of the particles after functionalization with oligopeptides

For the experiment shown in Figure 2, amorphous silica nanoparticles were treated with basic Piranha solution (5:1:1, H₂O/H₂O₂/NH₄OH) for 5 minutes at 80°C for cleaning the silica surfaces, rendering the particle surfaces more hydrophilic by surface hydroxylation, and increasing the number of silanol groups on the particles' surface. After extensive washing, using distilled water, the sample was air-dried and further reacted with thionyl chloride (SOCl₂) to convert the silanol groups into silyl chloride (Si-Cl) intermediates, using N,N-dimethylformamide (DMF) as catalyst. It has been reported that silyl chloride intermediates are easy susceptible to primary alcohol attack (Hergenrother PJ, Depew KM, Schreiber SL. Small-molecule microarrays: covalent attachment and screening of alcohol-containing small molecules on glass slides. JACS 122:7849-7850, 2000; Jiang W, Irgum K. Tentacle-type zwitterionic stationary phase prepared by surface-initiated graft polymerization of 3-[N,N-dimethyl-N-(methacryloyloxyethyl)-ammonium] propanesulfonate through peroxide groups tethered on porous silica. Anal Chem 74:4682-4687, 2002.). After treatment with tetrahydrofuran (THF) for 4 hours at room temperature, the amorphous silica particles with silyl chloride surface functionalization were incubated with the oligopeptide (EEEEEEEE; E₈) or the oligopeptide (SEEEEEEEE; SE₈) or the oligopeptide (SEEEEEEEE-Ahx-K-FITC; SE₈-FITC) overnight at room temperature. The N-terminal serine comprises a free hydroxyl group.. After washing, the samples were air dried and analyzed by Fourier transform infrared spectroscopy with attenuated total reflection (FTIR-ATR) and fluorescence microscopy.

Figure 2 shows the spectrum of non-functionalized amorphous silica particles within the range of 400 and 2000 cm⁻¹ (green spectrum; control 1). It displays the typical silica bands, i.e., a broad band located at 1106 cm⁻¹. This band is located in the wavelength ranging from ~1200 to 1100 cm⁻¹, related to Si-O-Si bridging and can be assigned to asymmetric stretching vibrations of Si-O-Si as observed in vitreous (amorphous) silica (Efimov AM. Optical Constants of Inorganic Glasses; CRC Press: New York, 1995). The broad band located at 1637 cm⁻¹ is attributed to the deformation vibrations of the O-H bond derived from both water molecules incorporated in the silica matrix and silanol groups.

Upon incubation of the silyl chloride functionalized amorphous silica nanoparticles with the oligonucleotide SE₈, the FTIR-ATR spectrum shows an additional shoulder at 980 cm⁻¹, which can be assigned to Si-O-C bonds (Grill A, Neumayer DA. J Appl Phys 94:6697, 2003; Grill A, Patel V. Appl Phys Lett 79:803, 2001; Landdry CJT, Coltrain BK, Wesson JA, Zumbulyadis N. Polymer 33:1496, 1992), indicating silica-organic material interaction, i.e., the oligopeptide is covalently bound to the amorphous silica surface by serine-derived hydroxyl attack to the silyl chloride intermediate (Figure 2, red spectrum). However, when the same particles are incubated with the oligopeptide E₈ (lacking the serine residue), no peak within this region can be observed (Figure 2, blue spectrum; control 2), indicating the requirement of a terminal hydroxylated amino acid residue for binding of the oligopeptide to the functionalized silica particles.

A typical FTIR-ATR spectrum of a protein contains peaks derived from the amide bonds vibrations, in particular the amide I band centered at ~1600-1700 cm⁻¹, which is largely due to C=O stretching with small contribution from the C-N stretch vibrations (Krimm S, Bandekar J. Adv Protein Chem 38:181-364, 1986; Haris PI, Severcan F. FTIR spectroscopic characterization of protein structure in aqueous and non-aqueous media. J Mol Catal B Enzym 7:207-221, 1999). The peak located at 1635 cm⁻¹ in the red spectrum can be attributed to the amide I band of SE₈, confirming again the presence of oligopeptides immobilized on the surface of the amorphous silica particles.

In parallel experiments, oligopeptides E₈ and SE₈ were employed, additionally comprising a C-terminal spacer (Ahx), followed by a lysine residue that was used to covalently bind FITC. The peptides were, then, incubated with silyl chloride functionalized amorphous silica nanoparticles overnight at room temperature. Afterwards, the samples were thoroughly washed, using distilled water, followed by a centrifugation step, in order to remove unbound oligopeptides. Finally, the samples were analyzed by fluorescent microscopy. As control, plain amorphous silica particles (not treated with thionyl chloride) were incubated with both FITC-labeled oligopeptides (overnight at room temperature) and thoroughly washed.

Figure 3 shows the fluorescence micrographs of amorphous silica particles following incubation with FITC-labeled oligopeptides (E₈-FITC and SE₈-FITC). Incubation of silyl chloride functionalized amorphous silica particles with SE₈-FITC elicited a strong fluorescent signal at the surface of the particles as a consequence of the covalent bond between silica surface and the hydroxyl moiety of the terminal serine (Figure 3D). Following incubation with E₈-FITC, however, only a weak fluorescence can be observed as a result of unspecific and non-covalent adsorption to the particles surface (Figure 3C). This result is very surprising as the carboxylic groups of the 8 glutamic acid residues were considered to be able to bind to the silyl chloride intermediates, similar to the hydroxyl group of the serine residue. However, these results corroborate the FTIR-ATR data (see above). On the other hand, when non-functionalized amorphous silica particles are incubated with E₈-FITC and SE₈-FITC overnight at room temperature, almost no fluorescent signal can be observed (Figure 3A,B), indicating the absence of silica surface-immobilized oligopeptides.

### Attachment of oligopeptide functionalized nanoparticles to tooth enamel

The immobilization of oligopeptide functionalized silica nanoparticles (20 nm) on tooth enamel and dentin can be studied by confocal laser scanning microscopy. The tooth surface was treated with silica nanoparticles, carrying FITC-labeled oligopeptides, for up to 1 hr at room temperature. After extensive washing with distilled water, the samples were inspected through confocal laser scanning microscopy. Figure 4A (cross section) and Figure 4B,C (longitudinal section) depict the controls that had been incubated with E₈-FITC, showing only weak (Figure 4A) or no fluorescence signals (Figure 4B,C) on the dental surface due to weak bonding between oligopeptide and silica nanoparticles (lack of serine residue). In Figure 4D-F the immobilization of silica nanoparticles (covalently functionalized with SE₈-FITC) on dental surfaces can be observed. The cross section shows dentinal tubules that had been filled with the tagged silica nanoparticles (Figure 4D). The longitudinal sections in Figure 4E and F depict both filled dentinal tubules and coated dental surface respectively. In parallel, electron microscopic inspection reveals a similar filling of dentinal tubules with the SE₈-FITC tagged silica nanoparticles (Figure 5B), whereas in the approach with E₈-FITC, the silica nanoparticles did not bind to the dental hydroxyapatite and, consequently, did not fill the dentinal tubules (Figure 5A).

### Attachment of oligopeptide functionalized microparticles to tooth enamel

Similar results have been obtained using silica microparticles with a diameter of 1 µm or larger. Figure 6 shows a scanning electron microscope (SEM) image of untagged silica microparticles with a diameter of 1 µm.

Following surface functionalization and immobilization of oligopeptides (e.g., with E₈ or SE₈), silica microparticles were incubated with slices of dental hydroxyapatite, similar to the approach using nanoparticles (see above). Figure 7B and C show the abundant binding of SE₈-carrying microparticles on dental hydroxyapatite. It is completely unexpected that the particles target the dentinal tubules' openings, where usually 1-3 particles assemble. There, the particles do not completely occlude the openings and do not enter the tubules. Rather they remain fixed at the tubules' openings. Hence, the permeability of the dentinal tubule system is not totally blocked and a limited exchange of small molecules is still possible. In the control sample, binding of E8-carrying microparticles rarely occurs (Figure 7A).

### Application of the oligopeptide-funtionalized nano-/microparticles as a carrier

A further aspect of this invention concerns oligopeptide-funtionalized nano-/microparticles, wherein the porous mineral is used as a carrier for bioactive compounds or dyes. Such bioactive compounds may comprise drugs (such as antiemetics, antibiotics, caffeine, nicotine), minerals and ions (such as fluoride), and vitamins that can be used for caries prophylaxis, therapy of periodontal diseases, dental care/-hygiene, or are of nutritive value. Additionally, flavoring agents (such as mint, cinnamon), or fluorescent dyes, titanium white, and other dyes might be used for cosmetic purposes (coloring/whitening of teeth). The preferred mineral used as a carrier for these additives are zeolites (aluminosilicates) due to their large surface area (high porosity), or periodic mesoporous organosilicas (PMOs; reviewed in: Hoffmann F, Cornelius M, Morell J, Fröba M. Periodic mesoporous organosilicas (PMOs): past, present, and future. J Nanosci Nanotechnol 6:265-288, 2006).

### Application of the oligopeptide-funtionalized nano-/microparticles as a supplement

The oligopeptide-funtionalized nano-/microparticles can be used as a supplement to tooth paste, chewing gum, dental silk, mouth rinse, or other products of dental care/- hygiene. The respective formulations can be prepared using state-of-the-art procedures.

### Methods

### Covalent binding of oligopeptides on functionalized nano-/microparticles

For covalent binding of oligopeptides, amorphous silica nano-/microparticles can be used (for example colloidal silica aqueous suspension). To initially assess their amorphous structure, silica nano-/microparticles are air-dried, ground to a thin powder, and analyzed by X-ray powder diffraction (XRD) in θ/2θ reflection geometry, using a diffractometer equipped with a position sensitive detector. The data is collected using Cu-Kα radiation at an operating potential of 40 kV and a current of 40 mA.

The results depicted in Figures 1-5 were obtained with particles with a diameter of 20 nm, but similar results have been obtained for particles with diameters of 1 µm and larger; Figure 6 and 7. The conversion of silanol groups to reactive silyl chloride can be performed according to state-of-the-art procedures (for example: Hergenrother PJ, Depew KM, Schreiber SL. JACS 122:7849-7850, 2000). In a modification of the protocol cited above, 100 mg amorphous silica nanoparticles (or microparticles) can be incubated, for example, with basic Piranha solution (H₂O/H₂O₂/NH₄OH; 5:1:1) for 5 min at 80°C to activate and hydroxylate surfaces. This procedure results in an increased number of silanol groups and, consequently, in increased surface hydrophilicity (Seu KJ, Pandey AP, Haque F, Proctor EA, Ribbe AE, Hovis JS. Biophys J 92:2445-2450, 2007). Afterwards, the samples are washed extensively with distilled water, pelleted (e.g., 5,000 x rpm, 10 min, room temperature), and air-dried. Then, silica particles (in the experiments shown: 50 mg) are re-suspended in a solution of dry tetrahydrofuran (THF) for 4 hr (room temperature). In the presence of 1% [v/v] SOCl₂ and 0.1% [v/v] DMF, silanol groups are converted into silyl chloride (Si-Cl), according to the reference cited above (Hergenrother PJ, Depew KM, Schreiber SL. JACS 122:7849-7850, 2000). Subsequently, the samples are pelleted again (e.g., 5,000 x rpm, 10 min, room temperature), washed in aqua dest. or THF, and air-dried.

Various oligopeptides can be used in the method described in this invention. In the examples shown, oligopeptides are used, which comprise the following amino acid sequences (single letter amino acid [aa] code) EEEEEEEE (E₈), SEEEEEEEE (SE₈), E₈-Ahx-K-FITC (E₈-FITC), or SE₈-Ahx-K-FITC (SE₈-FITC). 6-aminohexanoic acid (Ahx) is used as spacer, but also aminoalkanoic acids with other chain lengths (C=2 to C=16 or higher) can be applied. Lysine (K) provides the ε-amino group for labeling of peptides with fluorescein isothiocyanate (FITC), and serine (S) contributes the hydroxyl group required for covalent binding to the silyl chloride-functionalized nano-/microparticles. Peptide stock solutions can be prepared in dimethylsulfoxide (DMSO). Then, the respective oligopeptide (12 µg) is incubated overnight (room temperature) with the chloride-functionalized silica particles (2 mg) that have been re-suspended in DMSO (1 ml). Following extensive washing with aqua dest. and pelleting (e.g., 5000 x rpm, 10 min, RT), the FITC-labeled samples can be analyzed by fluorescence microscopy. Non-labeled samples can be analyzed by scanning electron microscopy or FTIR-ATR. Spectra are recorded at 4 cm⁻¹ resolution, averaging 32 scans. Peaks can be attributed according to: Vogel Al, Tatchell AR, Furnis BS, Hannaford AJ. Vogel's Textbook of Practical Organic Chemistry. Prentice Hall, Upper Saddle River, NJ, USA, 1998.

### Immobilization of oligopeptide functionalized nano-/microparticles to dental hydroxyapatite

Tooth enamel/dentin is incubated with hydrogen peroxide (30% [v/v]) for 24 h (room temperature) and subsequently washed with sodium hypochlorite solution (70% [v/v]) for 24 h (room temperature) to completely remove residual organic matter. Afterwards, the material is extensively washed with aqua dest. and air-dried for 24 h (70°C). Then, silica nano- or microparticles (0.2 mg) that have been functionalized, for example, with SE₈ or SE₈-FITC (1.2 µg in both cases) are resuspended in 1 ml phosphate buffered saline (PBS) and incubated with slices of the cleaned dental hydroxyapatite for up to 1 h (room temperature). Alternatively, whole teeth were immersed in 1 ml of the suspension for up to 1 h (room temperature). Surprisingly, in all cases, incubation time can be reduced to 5 min through prior etching of the dental hydroxyapatite with phosphoric acid. After washing in aqua dest., the samples are analyzed by fluorescence microscopy, confocal laser scanning microscopy (cLSM), or electron microscopy. In the latter case, the samples are mounted on carbon stubs and analyzed by scanning electron microscopy (SEM). Alternatively, the samples are transferred onto copper grids, coated with 1.2% Formvar, to conduct transmission electron microscopy (HRTEM). For cLSM analyses, the argon laser line of 488 nm is used to excite FITC.

## Claims

1. A nano-/microparticle functionalized with at least one hydroxyapatite-binding oligopeptide

2. The nano-/microparticle according to claim 1, wherein the nano-/microparticle consist of a mineral.

3. The nano-/microparticle according to claim 1 or 2, wherein the mineral consists of silica (amorphous silicon oxide), zeolite (microporous aluminosilicates), or periodic mesoporous organosilicas (PMOs).

4. The nano-/microparticle according to any of claims 1 to 3, wherein the oligopeptide consists of amino acids whose side chains carry one or two carboxylic acid functional groups, such as glutamic acid, aspartic acid, and/or gamma-carboxyglutamic acid.

5. The nano-/microparticle according to any of claims 1 to 3, wherein the oligopeptide consists of amino acids whose side chains carry an amino group or an amino group and a hydroxyl group such as lysine and hydroxylysine.

6. The nano-/microparticle according to any of claims 1 to 5, wherein the oligopeptide has a chain length of 3 to 18, preferably 8 amino acids and, in addition, a terminal serine residue.

7. The nano-/microparticle according to any of claims 1 to 6 wherein an aminoalkanoic acid with a chain length of 2 to 16 carbon residues, preferably 6-aminohexanoic acid (Ahx), is used as spacer.

8. The nano-/microparticle according to any of claims 1 to 4 and any of claims 6 and 7, wherein the oligopeptide comprises the following amino acid sequences SEEEEEEEE (SE₈) or SEEEEEEEE-Ahx-K(FITC) (SE₈-FITC), and wherein lysine (K) provides the ε-amino group for labeling of peptide with a fluorescent dye such as fluorescein isothiocyanate (FITC), and wherein the terminal serine residue (S) contributes the hydroxyl group required for covalent binding to silyl chloride-functionalized nano-/microparticles.

9. The nano-/microparticle according to any of claims 1 to 8, wherein the mineral carries bioactive compounds, such as drugs, such as, for example, antiemetics, antibiotics, caffeine, nicotine, minerals and ions such as, for example, fluoride, flavouring agents, such as, for example, mint, cinnamon, or vitamins, or fluorescent dyes, titanium white, and other dyes for coloring of teeth.

10. A tooth paste, chewing gum, dental silk, or mouth rinse, comprising the nano-/microparticle according to any of claims 1 to 9, preferably as a supplement.

11. A cosmetic method of sealing of pits, cavities, fissures, and dentinal tubules of teeth, comprising administering to a subject the nano-/microparticle according to any of claims 1 to 9, or the tooth paste, chewing gum, dental silk, or mouth rinse according to claim 10.

12. Use of the nano-/microparticle according to any of claims 1 to 9, for sealing of pits, cavities, fissures, and dentinal tubules of teeth in order to prevent caries formation and/or to reduce tooth hypersensitivity (dentin hypersensitivity).

13. Use according to claim 12, wherein the nano-/microparticles are contained in a tooth paste, chewing gum, dental silk, or mouth rinse.


SEQUENCE LISTING

<110> Müller, Werner E.G.
      Wiens, Matthias

<120> HYDROXYAPATITE-BINDING NANO- AND MICROPARTICLES FOR CARIES
      PROPHYLAXIS AND REDUCTION OF DENTAL HYPERSENSITIVITY

<130> FB23409

<160> 2

<170> PatentIn version 3.5

<210> 1
<211> 9
<212> PRT
<213> Artificial Sequence

<220>
<223> Oligopeptide

<400> 1

Ser Glu Glu Glu Glu Glu Glu Glu Glu
1               5


<210> 2
<211> 8
<212> PRT
<213> Artificial Sequence

<220>
<223> Oligopeptide

<400> 2

Glu Glu Glu Glu Glu Glu Glu Glu
1               5